# EUROPÄISCHE PATENTSCHRIFT

(11) **EP 0 456 792 B1**
(45) Veröffentlichungstag und Bekanntmachung des Hinweises auf die Patenterteilung: **13.10.1999**
(21) Anmeldenummer: 90917612.5
(22) Anmeldetag: 29.11.1990
(51) Int. Cl.: A61L 15/44, A61K 31/135

(54) **VERBESSERTES TRANSDERMALES SYSTEM ZUR APPLIKATION VON PHARMAKOLOGISCH AKTIVEN VERBINDUNGEN UNTER pH-KONTROLLIERTEN BEDINGUNGEN**
IMPROVED SYSTEM FOR THE TRANSDERMAL APPLICATION OF PHARMACOLOGICALLY ACTIVE COMPOUNDS AT CONTROLLED pH
SYSTEME TRANSDERMIQUE AMELIORE D'APPLICATION DE COMPOSES PHARMACOLOGIQUEMENT ACTIFS AVEC UN pH CONTROLE

(30) Priorität: 01.12.1989 DE 3939703
(43) Veröffentlichungstag der Anmeldung: 21.11.1991
(73) Patentinhaber: Boehringer Ingelheim International GmbH, 55216 Ingelheim (DE); Boehringer Ingelheim Pharma KG, 55216 Ingelheim am Rhein (DE)
(72) Erfinder: GUPTE, Arun, Rajaram, D-6507 Ingelheim (DE); ROHR, Uwe, D-6535 Gau-Algescheim (DE); ZIERENBERG, Bernd, D-6530 Bingen (DE)
(74) Vertreter: Laudien, Dieter, Dr.
(86) Internationale Anmeldenummer: EP9002052
(87) Internationale Veröffentlichungsnummer: WO9107998

(56) Entgegenhaltungen:
- EP-A- 0 197 504
- WO-A-87/00042
- DE-A- 3 843 557
- GB-A- 2 174 605
- US-A- 4 830 856

## Beschreibung

Die transdermale Applikation pharmakologisch aktiver Verbindungen unter Verwendung von transdermal therapeutischen Systemen ist seit längerem aus dem Stand der Technik bekannt. Geeignete Pflaster hierzu sind beispielsweise in den US-Patentschriften 3 558 122 und 3 558 123 offenbart. Obwohl in diesen und zahlreichen nachfolgenden Patentanmeldungen eine Vielzahl von Wirkstoffen als geeignet dargestellt werden, hat es sich in der Praxis gezeigt, daß es bei der Anwendung am Patienten auch dann zu unerwarteten Problemen kommen kann, wenn durch in vitro Versuche bereits nachgewiesen werden konnte, daß das wirkstoffhaltige Pflaster den Wirkstoff in ausreichender Menge abgibt.

Dagegen konnten bei der Übertragung auf den Menschen jedoch nicht die erwarteten ausreichend hohen oder konstanten Blutspiegelwerte erzielt werden. So wird beispielsweise Clenbuterol oral in Form von Tabletten oder Säften als β-Sympathomimetikum zur Behandlung von Asthma bronchiale eingesetzt. Entgegen den Erwartungen führte die transdermale Applikation von Clenbuterol bislang nicht zu konstanten Blutspiegelwerten.

Überraschenderweise wurde nun festgestellt, daß bei einer transdermalen Applikation von Clenbuterol die Fluxrate für den Wirkstoff entscheidend verbessert werden kann, wenn der pH-Wert der Hautoberfläche auf einen Wert konstant zwischen 6,0 und 8,5 gehalten wird.

Die Möglichkeit, auf den pH-Wert einer wirkstoffhaltigen Lösung innerhalb eines Wirkstofffreigabesystems Einfluß zu nehmen, ist zwar aus dem Stand der Technik bekannt [US-A-4,830,856], jedoch bestanden bisher keine Erkenntnisse, auf welche Weise über den pH-Wert die Fluxrate des Wirkstoffs erhöht werden kann.

Die Fluxrate eines Wirkstoffes durch die Haut ist definiert als Menge des Wirkstoffes pro Hautoberfläche und pro Zeiteinheit und wird üblicherweise in [µg/cm² . h] angegeben. In dem oben angegebenen pH-Bereich erreicht die Fluxrate für Clenbuterol ihr Maximum, während bei höheren und niederen pH-Werten die Fluxrate stark abnimmt.

Der pH-Wert der Hautoberfläche ist beim Menschen individuell und temporär verschieden. Ein als Standard angenommener pH-Wert von 515 bis 6,0 schwankt im Individualfall zwischen pH 4,5 und 8,0 und hängt von vielen unterschiedlichen Faktoren ab. Schwankungen des pH-Wertes können daneben zum einen durch das Pflaster selbst und zum anderen durch den Wirkstoff hervorgerufen werden. Wie aus der Abb. I ersichtlich ist, nimmt die Fluxrate von Clenbuterol außerhalb des angegebenen pH-Bereichs drastisch ab, so daß eine ausreichende Permeabilität der Haut für Clenbuterol nicht mehr gewährleistet ist.

Die Erfindung betrifft die Verwendung von schwachen Basen, schwachen Säuren organischen und anorganischen Salzen, die mit der Hautoberfläche ein Puffersystem bilden, oder von Puffergemischen in einem wirkstoffhaltigen transdermalen therapeutischen System (TTS), das eine wirkstoffundurchlässige Rückschicht, ein wirkstoffhaltiges Reservoir, Mittel zur Befestigung auf der Haut und gegebenenfalls eine Membran zur Steuerung der Wirkstofffreigabe enthält, zur Einstellung des pH-Wertes der Hautoberfläche in einem Bereich der der maximalen Fluxrate des o.g. Wirkstoffs entspricht. Erfindungsgemäß wird dadurch bei Patienten unabhängig vom Eigen-pH-Wert der Haut die optimale Fluxrate für den Wirkstoff eingestellt.

In besagten transdermalen Systemen kann der Zusatzstoff beispielsweise auf der Seite angebracht sein, die mit der Haut in Kontakt kommt, so daß die Hautoberfläche in dem gewünschten pH-Bereich gepuffert wird. Es ist selbstverständlich daß die dazugehörigen Substanzen oder Substanzgemische pharmakologisch verträglich sein müssen. Für die Einstellung eines vorher bestimmbaren pH-Wertes auf der Haut sind Zusatzstoffe wie z.B. schwache Basen, schwache Säuren, organische und anorganische Salze, die mit der Hautoberfläche ein Puffersystem bilden oder Puffergemische (Puffersysteme) geeignet.

Die Menge des Zusatzstoffes darf nicht zu gering sein, damit sichergestellt ist, daß der pH-Wert auf der Hautoberfläche auch für die gesamte Tragedauer des Pflasters auf den gewünschten Wert eingestellt werden kann.

Die Menge des Zusatzstoffes beträgt im allgemeinen zwischen 2 und 10 Gew.-% bezogen auf das Gewicht des Wirkstoffreservoirs des transdermalen Systems, bevorzugt ist ein Bereich zwischen 4 und 6 Gew.-%.

Diese Menge an Zusatzstoff ist ausreichend um den pH der Haut über einen Zeitraum von 1 bis 7 Tagen auf einen vorherbestimmten Wert einzustellen.

Vorteile einer solchen pH-Wert-Einstellung sind einmal, daß basische Wirkstoffe, die auf der Hautoberfläche einen basichen pH-Wert einstellen, durch Zusatz von sauren Salzen den Haut-pH-Wert in den sauren Bereich verschieben können. Dadurch kann die Verträglichkeit von transdermalen Systemen erheblich verbessert werden, da das Bakterienwachstum im sauren pH-Wert-Bereich erheblich eingeschränkt ist.

Ein anderer Vorteil der pH-Wert-Einstellung auf der Hautoberfläche ist, daß schwach saure bzw. basische Wirkstoffe durch Zusatz von geeigneten Salzen ein Puffersystem gebildet wird und sich ein definierter pH-Wert einstellt, so daß Schwankungen im Hautoberflächen pH-Wert dadurch ausgeglichen werden können.

Geeignete Salze oder schwache Säuren, die sich als Zusatzstoff zur pH-Wert-Einstellung eignen, sind beispielsweise folgende:

Dinatriumhydrogenphosphat, Natriumdihydrogenphosphat, Natriumacetat, Kaliumdihydrogenphosphat, Natriumcarbonat, Natriumhydrogencarbonat, Borsäure, Natriumborat, Citronensäure, Natrium- oder Kaliumcitrat, Monocalciumorthophosphat (Ca(H₂PO₄)₂), Kaliumhydrogenphosphat, Dikaliumhydrogenphosphat, Weinsäure, Kalium oder Natriumtartrat, Natriumhydrogenphthalat.

Puffersysteme und Puffergemische, mit denen ein pH-Wert zwischen 3 und 10 eingestellt werden kann, sind aus dem Stand der Technik hinreichend bekannt.

Im folgenden werden Beispiele für Wirkstoffe genannt, die durch eine pH-Wert-Einstellung in ihrem Diffusionsverhalten geändert werden können:

Physostigmin, Clonidin, Fentanyl, MR 2264 (N-(2-Methoxyethyl)-noroxy-morphon), Ephedrin, Nicotinsäureamid, Clenbuterol, Pramipexol, Lisurid, Terbutalin, Salbutamol, Hexoprenalin, Insulin, Vasopressin, Atriales Natriuretisches Peptid (ANP).

Die erfindungsgemäßen Vorteile und Befunde lassen sich auch auf andere Wirkstoffe übertragen, die in Form schwacher Basen oder schwacher Säuren vorliegen.

Das erfindungsgemäße Pflaster kann auch vorteilhaft genutzt werden, um den pH-Wert der Hautoberfläche leicht sauer (pH = 5,5 bis 6,9) einzustellen, um auf diese Art unerwünschtes Bakterienwachstum und eventuell damit verbundene Hautreizungen zu vermeiden.

Besonders geeignet hierfür sind neutrale Moleküle, deren Fluxrate nicht oder nur gering durch den pH-Wert der Haut beeinflußt wird, wie z.B. Nitroglycerin.

Transdermale Systeme, die sich für den erfindungsgemäßen Zusatz zur Einstellung des pH-Wertes auf der Haut eignen, sind aus dem Stand der Technik bekannt. Im allgemeinen sind dies Matrixsysteme aus einem der nachfolgend genannten Polymere oder Copolymere.

Polymethacrylat, Polyvinylpyrrolidon, Ethylzellulose, Hydroxypropylmethylzellulosephthalat, Polyvenylalkohol bzw. deren Copolymerisate mit Vinyllaurat oder Maleinsäure, Vinylacetat bzw. dessen Copolymerisat mit Vinyllaurat, oder Maleinsäure, Vinylacetat bzw. dessen Copolymerisat mit Vinyllaurat oder Maleinsäure, Polyvinylether, Butylkautschuk und Polycaprolactam.

Bevorzugte Polymere und Copolymere sind solche, die durch Emulsionspolymerisation hergestellt wurden. Bei solchen Polymerisaten ist bekannt, daß die Wirkstofffreisetzung durch Variation der Teilchengröße der Polymerteilchen durch Variation der Schichtdicke im Bereich zwischen 40 und 200 µm, bevorzugt bis 140 µm und durch Variation der Glastemperatur eingestellt werden kann.

Die Teilchengröße bezieht sich auf den Teilchendurchmesser des polymeren Materials nach dessen Herstellung und kann bis 500 µm betragen. Die Teilchengröße (Durchmesser) kann in Abhängigkeit von den Polymerisationsbedingungen eingestellt werden. Eine Abnahme der Teilchengröße bewirkt eine Erhöhung der Freisetzungsrate.

Die Glastemperatur kann durch Änderung der Monomerzusammensetzung eingestellt werden und liegt beispielsweise zwischen -20° und + 80°C, bevorzugt zwischen - 20° und + 40°C, besonders bevorzugt zwischen -10° und + 30°C. Ein Anstieg der Glastemperatur ist mit einer Erniedrigung der Freisetzungsrate verbunden.

Nach dem Emulsionspolymeriationsverfahren lassen sich beispielsweise folgende Polymere herstellen, wie z.B. PVC, Polylactide, Polystyrol, Polyvinylacetat, Polybutadien, Polyacrylnitril, Polyvinylester, Polyvinylether und deren Copolymere. Bevorzugt sind emulsionspolymerisierte Copolymerisate von Methyl- und/oder Ethylestern der Acryl- und Methacrylsäure. Das Molekulargewicht der Emulsionspolymere sollte zwischen 10⁴ und 10⁷ betragen. Die Gewinnung des Trägermaterials als Feststoff kann z.B. durch Gefriertrocknung erfolgen, hierbei bleiben die Polymerisatteilchen in ihrer Form und Größe erhalten.

Matrixsysteme für die transdermale Applikation bestehen aus einer wirkstoffhaltigen Rückschicht, einem wirkstoffhaltigen Reservoir (Wirkstoffmatrixdepot) und Mitteln zur Befestigung auf der Haut.

Die Steuerung der Wirkstofffreigabe erfolgt entweder durch geeignete Auswahl der Polymermatrix - wie z.B. in der europäischen Patentschrift 86 997 offenbart - oder aber durch geeignete Membranen, wie zum Beispiel in den US-Patentschriften 3 598 122 und 3 598-123 beschrieben.

In einer Ausführungsform enthält das System eine wirkstoff-undurchlässige Rückschicht, eine wirkstofhaltige Polymermatrix mit 2 bis 10 Gew.-% einer schwachen Base, einer schwachen Säure oder eines Salzes zur Einstellung des pH-Wertes auf der Haut sowie Mittel zur Befestigung des Systems auf der Haut.

Bevorzugt ist eine wirkstoffhaltige Matrix aus einem emulsionspolymerisierten Polyacrylat. Solche Systeme sind beispielsweise in der deutschen Offenlegungsschrift 2 920 500, der europäischen Patentanmeldung 209 121 und der europäischen Patentschrift 86 997 offenbart, auf die hier mit inhaltlich Bezug genommen wird.

Besonders bevorzugte Emulsionspolymerisate sind die Copolymerisate auf der Basis der Alkylester der Acryl- und Methacrylsäure. Die allgemeine Formel ist 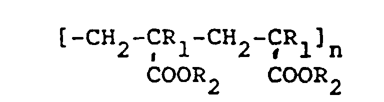
worin R₁ = H, CH₃ und R₂ = H, C₁-C₄-Alkyl C₁-C₄-Alkyl-N(C₁-C₄-Alkyl)₂ bedeutet.

Das mittlere Molekulargewicht liegt zwischen 6 . 10⁴ und 1,6 - 10⁷, bevorzugt ist ein Bereich zwischen 10⁴ und 10⁶.

Bevorzugt sind die Emulsionspolymerisate Eudragit

| | | | |
|---|---|---|---|
| E 30 D | MG | 800 000 | R₁ = H, CH₃, |
| | | | R₂ = CH₃C₂H₅; |
| E 12,5/100 | MG | 150 000 | R₁ = CH₃ |
| | | | R₂ = CH₂-CH₂-N(CH₃)₂ CH₃, C₄H₉; |
| L/S 100 | MG | 135 000 | R₁ = CH₃ |
| | | | R₂ = H, CH₃; |

der Firma Röhm, Darmstadt sowie Mischungen davon.

Zur Herstellung der oben beschriebenen Ausführungsform mit einer Matrix aus einem Emulsionspolymerisat wird wie folgt vorgegangen:

Der gefriergetrocknete Latex wird in einem organischen Lösungsmittel oder Lösungsmittelgemisch aufgenommen, das sowohl den Arzneistoff als auch das Polyacrylat zu lösen vermag. Der Zusatzstoff zur Einstellung des pH-Wertes auf der Haut wird entweder in fein verteilter Form als Pulver zugesetzt, oder falls das Lösunsmittel mit Wasser mischbar ist, auch in Form einer Lösung oder Suspension in Wasser. Als Lösungsmittel kommen niedere aliphatische Alkohole, Äther, Ketone, Ester, Kohlenwasserstoffe bzw. Halogenkohlenwasserstoffe in Frage, insbesondere solche, deren Siedepunkt unter 100°C liegen und die sich leicht verdampfen lassen. Auch Lösungsmittelgemische sind verwendbar. Durch Wahl eines geeigneten Lösungsmittels oder Lösungsmittelgemisches lassen sich die Viskositäten der Ausgangslösung verändern. Die Filme sollen normalerweise eine Dicke von etwa 50 bis 200 µm besitzen. Die Temperatur, bei der die Trocknung der Lösung zu einem Film erfolgt, liegt normalerweise bei Raumtemperatur bis maximal zur Siedetemperatur des verwendeten Lösungsmittel oder Lösungsmittelgemisches, wobei man normalerweise versuchen wird, wegen der Zersetzlichkeit vieler Arzneiwirkstoffe und der Gefahr der Blasenbildung im Film die Trocknung bei möglichst tiefen Temperaturen vorzunehmen. Die Filmherstellung kann kontinuierlich oder diskontinuierlich erfolgen. Die erhaltenen Filme werden in geeignete Stücke oder Stanzlinge zerschnitten und in üblicher Weise zur Herstellung von transdermalen Applikationsformen konfektioniert, etwa indem auf der einen Seite des wirkstoffhaltigen Films eine Stütz- und/oder Deckschicht und auf der anderen Seite eine Klebstoffschicht mit abziehbarem Schutzüberzug aufgebracht wird. Die Befestigung auf der Haut kann auch unter Verwendung eines klebenden Deckpflasters erfolgen.

In einer anderen Ausführungsform, bei der das transdermale System eine Membran zur Steuerung der Wirkstofffreigabe enthält, befinden sich die Zusatzstoffe zur Einstellung des pH-Wertes auf bzw. in der Haut zugewandten Seite der Membran.

In einer weiteren Ausführung ist das transdermale System als Mehrkammersystem aufgebaut, wobei in diskreten Kammern ein oder mehrere Wirkstoffe inkorporiert sind und in anderen Kammern erfindungsgemäß sich die Zusatzstoffe zur Einstellung des pH-Wertes befinden. Die Zusatzstoffe können in diskreten Kammern, beispielsweise in Form eines Gels, einer Lösung oder einer Suspension vorliegen.

Unabhängig von der Art des Matrixsystems kann der Zusatzstoff zur Einstellung des pH-Wertes in einer separaten Schicht auf der Haut zugewandten Seite enthalten sein. Diese Schicht kann in Form eines klebrigen Polymers, eines nichtklebrigen Polymers eines Gels, z.B. eines Agarosegels, in Form einer (viskosen) Lösung oder in Form kleiner Partikel vorliegen. Es ist hierbei nicht unbedingt errforderlich, daß die separate Schicht mit dem Zusatzstoff die Wirkstoffmatrix ganzflächig bedeckt.

In einer anderen Ausführungsform enthält das transdermale System auf der der Haut zugewandten Seite kleine Nadeln, die das Stratum Corneum durchdringen und somit Arzneistoffen die ungehinderte Diffusion durch diese Nadelstiche ermöglicht. (Typ: Moskitosystem). Die benötigten Salze halten die Wirkstoffe einmal in Lösung, so daß sie ohne auszukristallisieren durch das Stratum Corneum in die Epidermis dringen können, aber verhindern auch durch Wahl eines günstigen pH-Wertes - im sauer gepufferten Bereich - das Bakterienwachstum, so daß Konservierungsstoffe auf der Haut vermieden werden können, die zu Unverträglichkeitsraktionen führen können. Transdermal anzuwendende Pflaster dieses Typs sind beispielsweise in der DE-OS 23 05 989 beschrieben.

### Beschreibung der Figuren:

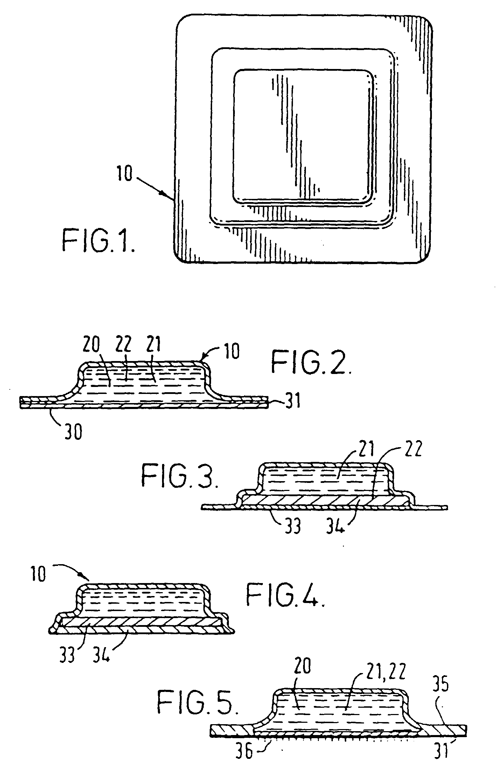

Figur 1 der Zeichnungen zeigt die Aufsicht auf ein Pflaster 1. Abweichend von der dargestellten Zeichnung kann das Pflaster ebensogut rechteckig oder kreisförmig ausgebildet sein.
Figur 2 zeigt einen Querschnitt durch eine bevorzugte Ausführungsform des Pflasters 10, bei der der Wirkstoff 21 und der Zusatzstoff 22 zur Einstellung des pH-Wertes in einer Polymermatrix gleichförmig verteilt ist. Die Schutzfolie 30 wird von der Anwendung abgezogen, so daß die Klebefläche 31 freigeiegt wird.
Figur 3 zeigt eine andere Ausführungsform des Pflasters 10, bei der die Wirkstofffreigabe durch eine Membran 33 gesteuert wird. Der Zusatzstoff 22 ist in einer separaten Schicht 34 enthalten. In dieser Abbildung ist die Schutzfolie 30 nicht dargstellt.
Figur 4 zeigt eine ähnliche Ausführungsform des Pflasters 10, jedoch besteht die Schicht 34 aus einem klebrigen Polymer, das den Zusatzstoff 22 enthält.
Figur 5 zeigt ein Querschnitt durch ein Pflaster, das an seiner Unterseite kleine Nädelchen 36 zum Durchdringen der obersten Hautschicht aufweist. Die Polymermatrix 20 enthält den Wirkstoff 21 sowie den Zustzstoff 22. Die abgeflachten Außenseiten (35) des Pflasters enthalten auf der Unterseite eine klebende Schicht (31) zur Befestigung auf der Haut.

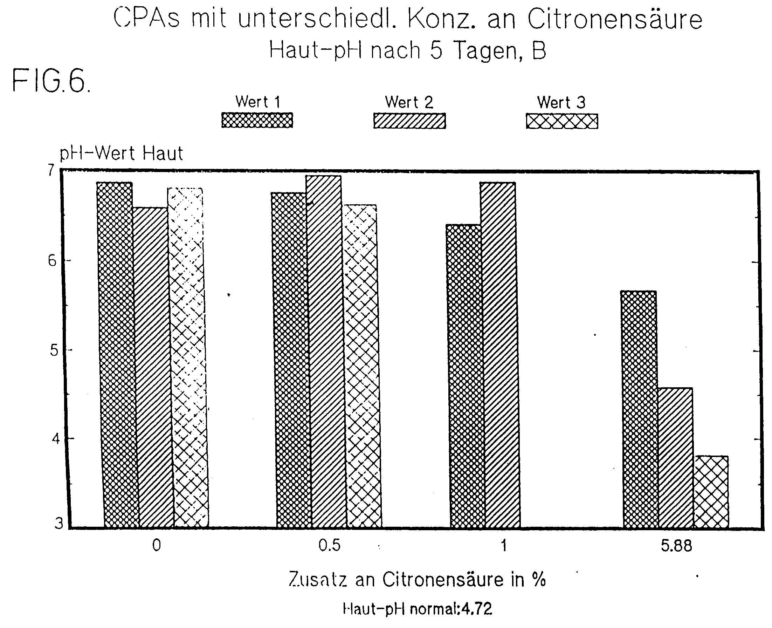

Auf die Figuren 6, 7 und 8 wird in den nachfolgenden Beispielen näher eingegangen.

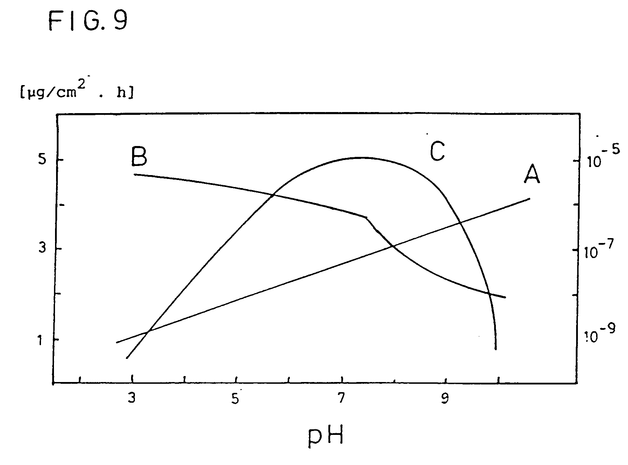

In Fig. 9 ist die Permeabilität P von Clenbuterol durch die menschliche Haut aufgetragen (Funktion A); P ist angegeben in [cm/sec]. Die Kurve B beschreibt die Wasserlöslichkeit der Substanz in Abhängigkeit vom pH-Wert [mg/ml]. Die Kurve C gibt die Fluxrate von Clenbuterol über einen weiteren pH-Wert-Bereich an. (Die rechte Ordinate in Fig. 9 ist logarithmisch dargestellt.

Die nachfolgenden Beispiele sollen die Erfindung näher erläutern.

### Herstellungsbeispiel 1

Herstellng der Clenbuterol - Eudragit® NE 30 D Lösung:

In einem beheizbaren und luftdicht verschließbaren Behälter wird unter Rühren

| | |
|---|---|
| Aceton | 1734 mg |
| vorgelegt und unter Rühren | |
| Clenbuterol | 21 mg |
| sowie | |
| Citronensäure | 21 mg |
| langsam hinzugefügt. Dann erfolgt die Zugabe von | |
| Eudragit® NE 30 D | 434 mg |

Der Behälter wird geschlossen und unter Rühren auf 40°C erwärmt. Bei dieser Temperatur wird gerührt bis eine homogene Lösung entstanden ist. Die Lösung muß klumpfrei sein. Die Viskosität der Lösung soll zwischen 3000 und 4000 mPas liegen. Die erwärmte Lösung wird mit einer Direktbeschichtungsanlage, besehend aus Auftragseinrichtung, Heizkanal und Kühlung aufgebracht. Quer zur Laufrichtung ist bei der Auftragseinrichtung ein feststehendes Messer (Rakel) angebracht.

Die oben beschriebene hergetellte viskose acetonische Lösung wird mit Hilfe des Rakels auf eine Trägerfolie aufgebracht.

Die Folienherstellung erfolgt somit einem Verfahren, wie es auch unter "Technologische Schriftenreihe: Veredelung bahnförmiger Materialien, Beschichten und Imprägnieren," Berger Verlag, Frankfurt, beschrieben ist.

Das Aceton des gegossenen Filmes wird entweder durch die Raumtemperatur oder durch einen Heizkanal verdampft. Die beschichtete Trägerbahn wird nach Abkühlen auf eine Folie aufgewickelt.
Stanzlinge in beliebiger Größe können ausgestanzt werden. Die Stanzlinge werden in ein Deckpflaster eingeklebt und können dann beim Patienten aufgeklebt werden.

### Herstellungsbeispiel 2

Herstellung der Clenbuterol - Eudragit® NE 30 D Lösung:

In einem beheizbaren und luftdicht verschließbaren Behälter wird unter Rühren

| | |
|---|---|
| Aceton | 1734 mg |
| vorgelegt und unter Rühren | |
| Clenbuterol | 21 mg |
| sowie | |
| Natriumcarbonat | 21 mg |
| langsam hinzugefügt. Dann erfolgt die Zugabe von | |
| Eudragit® NE 30 D | 434 mg |

Die weitere Verarbeitung erfolgt wie in Herstellungsbeispiel 1 beschrieben, jedoch mit dem Unterschied, daß anstelle einer klaren Lösung eine Suspension entsteht.

### Beispiel 1

Permeabilität von pH-Wort modifiziertem Clenbuterol CPA durch Humanhaut:

Zusammensetzung der CPA's:

| | pH 10.0 | pH 3.5 |
|---|---|---|
| Clenbuterol | 5 % | 5 % |
| Zitronensäure | | 5 % |
| Na₂CO₃ | 5 % | |
| Polymethacrylat Eudragit® E 30 D | 90 % | 90 % |

Als Freigabeapparatur wurde eine Franz-Zelle verwendet. Dies ist eine übliche Methode, um die Abgabe von Arzneistoffen aus pharmazeutischen Formulierungen zu testen. Proben wurden nach 24 h bzw. 48h entnommen und der Gehalt an Clenbuterol bestimmt.

| | pH-Wert 3.5 | | | pH-Wert 10.0 | | |
|---|---|---|---|---|---|---|
| Zelle | 1 | 2 | 3 | 1 | 2 | 3 |
| Clenbuterol Diffusion nach 24 h in µg/cm² | 3.48 | 0.65 | 2.64 | 7.8 | 11.44 | 9.84 |
| Clenbuterol Diffusion nach 48 h in µg/cm² | 5.52 | 1.69 | 6.04 | 21.7 | 27.3 | 24.2 |

Man erkennt deutlich, daß bei einem alkalischen pH-Wert eine höhere Diffusionsrate bestimmt wurde, als bei saurem pH-Wert.

### Beispiel 2

Figur 6 zeigt Ergebnisse bezüglich des HautoberflächenpH-Wertes, die unter dem System auf der Haut nach 5 Tagen Tragezeit mit Hilfe einer Oberflächen pH-Wert-Elektrode bestimmt wurden. Die getesteten Systeme unterscheiden sich durch ihre Konzentration an Citronensäure in der Polymethacrylatmatrix. Es ist deutlich zu erkennen, daß erst eine Citronensäurekonzentration höher als 1 % eine merkliche Veränderung des Oberflächen-pH-Wertes der Haut bewirkt. Mit "Wert 1,2 und 3" sind Probanden gemeint.

### Beispiel 3

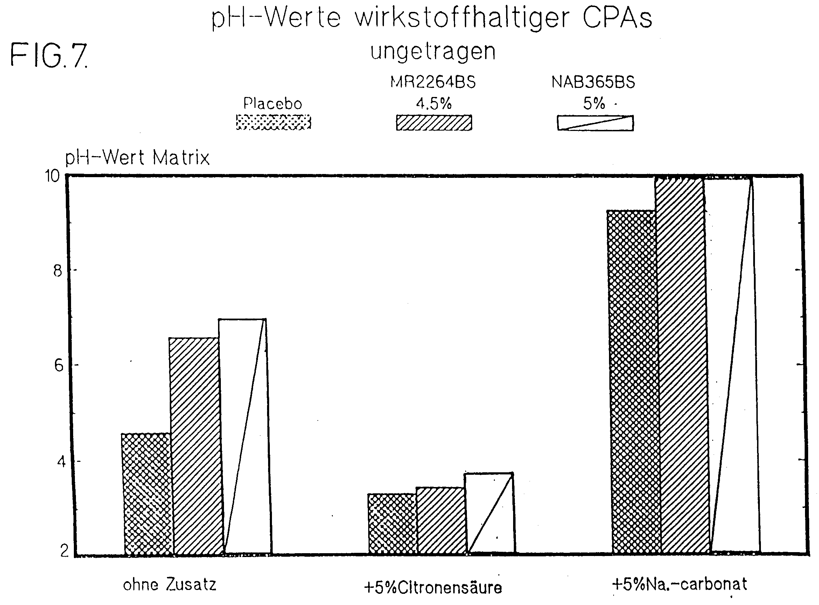

Fig. 7 zeigt Oberflächen-pH-Werte von wirkstoffhaltigen CPA's von basichen Wirkstoffen, die durch Zusatz von 5 % Citronensäure oder von Na₂CO₃ verändert wurden. Als Vergleich wurde auch der Oberflächen pH-Wert bestimmt, wie er ohne Zusatz von Salzen oder ionischen Stoffen vorliegt. Deutlich erkennt man, daß der pH-Wert veränderbar ist, sowohl zu basichen pH-Werten, als auch zu sauren pH-Werten.

### Beispiel 4

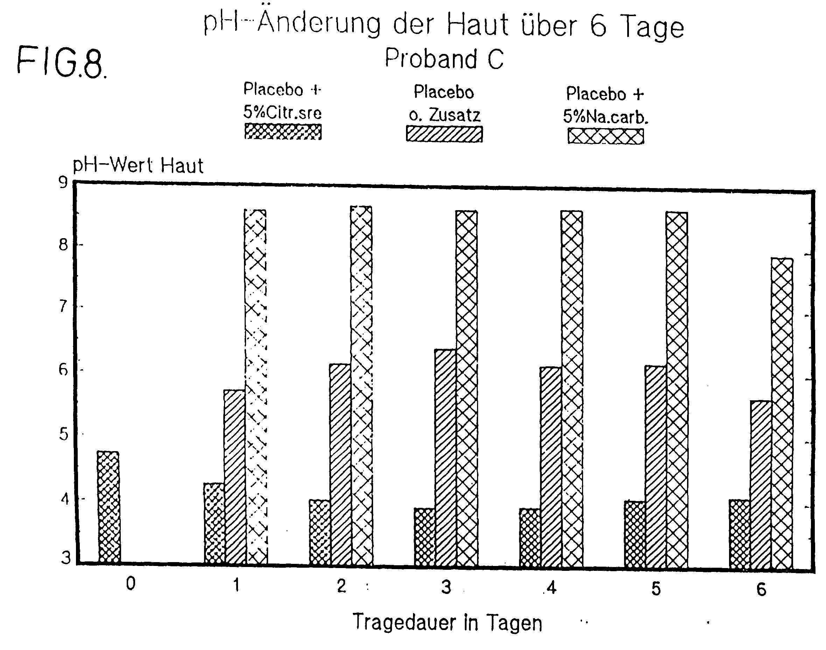

Fig. 8 zeigt den pH-Wert auf der Haut unter einem applizierten transdermalen therapeutischen Systems während 6 Tagen Tragedauer. Deutlich sind die pH-Wert-Einstellungen auf der Oberfläche zu erkennen. Der eingestellte pH-Wert wird während dieser Zeit auf der Haut beibehalten.

## Patentansprüche

1. Verwendung von schwachen Basen, schwachen Säuren, organischen und anorganischen Salzen, die mit der Hautoberfläche ein Puffersystem bilden oder von Puffergemischen als Zusatzstoff in einem wirkstoffhaltigen transdermalen therapeutischen System (TTS), das eine wirkstoffundurchlässige Rückschicht, ein wirkstoffhaltiges Reservoir, Mittel zur Befestigung auf der Haut und gegebenenfalls eine Membran zur Steuerung der Wirkstofffreigabe enthält, zur Einstellung des pH-Wertes der Hautoberfläche in einem Bereich der der maximalen Fluxrate [µg/cm²h] des oben genannten Wirkstoffs entspricht.

2. Verwendung nach Anspruch 1, dadurch gekennzeichnet, daß der Wirkstoff und der Zusatzstoff in einer Polymermatrix, bevorzugt aus einem emulsionspolymerisierten Polyacrylat, inkorporiert ist.

3. Verwendung nach einem der Ansprüche 1 oder 2, dadurch gekennzeichnet, daß die Menge des Zusatzstoffes zwischen 2 und 10 Gew.-% bezogen auf das Gewicht der wirkstoffhaltigen Polymermatrix beträgt.

4. Verwendung nach einem der Ansprüche 1 bis 3, dadurch gekennzeichnet, daß der Wirkstoff Clenbuterol ist.

## Claims

1. Use of weak bases, weak acids, organic and inorganic salts which form a buffer system with the surface of the skin or of buffer mixtures as additives in a transdermal therapeutic system (TTS) containing an active substance, said system containing a backing layer impervious to the active substance, a reservoir of active substance, means of attachment to the skin and optionally a membrane for controlling the release of active substance, for adjusting the pH of the skin surface within a range which corresponds to the maximum flow rate [µg/cm²h] of the above-mentioned active substance.

2. Use according to claim 1, characterised in that the active substance and the additive are incorporated in a polymer matrix, preferably consisting of an emulsion-polymerised polyacrylate.

3. Use according to one of claims 1 and 2, characterised in that the quantity of additive is between 2 and 10 % by weight, based on the weight of the polymer matrix which contains the active substance.

4. Use according to one of claims 1 to 3, characterised in that the active substance is clenbuterol.

## Revendications

1. Utilisation de bases faibles, d'acides faibles, de sels organiques et inorganiques, qui forment un système tampon avec la surface de la peau ou de mélanges tampons comme additif dans un système thérapeutique transdermique (STT) contenant un principe actif, qui contient une couche dorsale imperméable au principe actif, un réservoir contenant le principe actif, des moyens pour la fixation sur la peau et éventuellement une membrane pour la commande de la libération du principe actif, pour régler le pH de la surface de la peau dans un domaine qui correspond au flux maximal [µg/cm²h] du principe actif cité ci-dessus.

2. Utilisation selon la revendication 1 caractérisée en ce que le principe actif et l'additif sont incorporés dans une matrice polymère, de préférence constituée par un polyacrylate polymérisé en émulsion.

3. Utilisation selon l'une des revendications 1 et 2 caractérisée en ce que la quantité de l'additif est située entre 2 et 10%-en masse par rapport à la masse de la matrice polymère contenant le principe actif.

4. Utilisation selon l'une des revendications 1 à 3 caractérisée en ce que le principe actif est le clenbutérol.
